# EUROPEAN PATENT APPLICATION

(11) **EP 4 285 878 A1**
(43) Date of publication of application: **06.12.2023**
(21) Application number: 23174754.4
(22) Date of filing: 23.05.2023
(51) Int. Cl.: A61F 9/008

(54) **DEVICE AND METHOD FOR MONITORING OPHTHALMOLOGICAL LASER TREATMENT DEVICE**

(30) Priority: 01.06.2022 CH 6692022
(71) Applicant: Ziemer Ophthalmic Systems AG, 2562 Port (CH)
(72) Inventor: Rathjen, Christian, 28197 Bremen (DE); Steinlechner, Michael, 8006 Zürich (CH)
(74) Representative: Rentsch Partner AG

(57) **Abstract**

An ophthalmological laser treatment device (1) and method for controlling an ophthalmological laser treatment device (1) are disclosed, the device (1) comprising a base station (2) which has a treatment laser source (21) configured to generate a treatment laser beam, a control module (3), an application head (4), and an arm (5) arranged between the base station (2) and the application head (4), the application head (4) including a primary laser beam monitor (6) which is retractable out of the treatment laser beam and a secondary laser beam monitor (7).

## Description

### FIELD OF THE DISCLOSURE

The present invention relates to a device and method for monitoring an ophthalmological laser treatment device.

### BACKGROUND OF THE DISCLOSURE

Ophthalmological treatment devices, which use a laser for eye treatment, are known. The ophthalmological treatment device has a laser source, which produces a pulsed laser beam. Additionally, the wavelength of the laser light produced by the ophthalmological treatment device is dependent on the type of eye treatment and is typically in the ultraviolet (190nm to 230nm) or infrared (780nm to 1100nm) range.

The laser beam is typically produced by a laser source arranged in a base station. The laser beam is then guided along a beam path to an application head, where the laser beam is focused onto a patient's eye.

For correct and safe treatment, it is important to ensure that the laser beam is focused onto the correct point, in particular at intended locations on or in the eye. Depending on the specific implementation of the ophthalmological laser treatment device, this can be challenging, in particular if the application head is movable and therefore the beam path is not absolutely static. Additional sources of error include thermal drift and mechanical tolerances.

In particular, where the application head is connected to the base station using an arm, for example a rotatable, telescopic, or articulated arm, and the beam path runs through the arm, movement of joints in the arm can result in changes in the characteristics of the laser beam as it reaches the application head and ultimately the patient's eye.

EP3364924B1 teaches an automatic calibration of a treatment laser using an external grid target that is placed into the beam path at a precisely defined position external to the treatment device, corresponding to the position that a patient's eye will have during treatment. A disadvantage of this method includes the requirement of manually placing the grid target into the beam path.

DE 1 02019124164A1 teaches a laser treatment system and method for characterizing a laser beam whereby the energy and/or position of a pulsed treatment laser beam is intermittently guided onto a sensor.

US9592156B2 discloses a power detector used to monitor the power level of a laser beam in an ophthalmological surgery apparatus.

### SUMMARY OF THE DISCLOSURE

It is an object of the invention and embodiments disclosed herein to provide a device and method for monitoring an ophthalmological laser treatment device.

In particular, it is an object of the invention and embodiments disclosed herein to provide an ophthalmological laser treatment device and method for monitoring an ophthalmological laser treatment device which does not have at least some of the disadvantages of the prior art.

The present disclosure relates to an ophthalmological laser treatment device comprising a base station having a treatment laser source configured to generate a treatment laser beam, a control module, and an application head. An arm is arranged between the base station and the application head configured to provide a beam path for the treatment laser beam. The application head includes a primary laser beam monitor which is retractable out of the treatment laser beam and a secondary laser beam monitor. The control module is configured to move the primary laser beam monitor into the treatment laser beam. The control module is configured to receive a primary signal from the primary laser beam monitor and a secondary signal from the secondary laser beam monitor. The control module is configured to determine, using the primary signal, primary signal characteristics and determine, using the secondary signal, secondary signal characteristics. The control module is configured to determine whether one or more of the primary signal characteristics and one or more of the secondary signal characteristics satisfy one or more pre-defined tolerance limits. The control module is configured to retract the primary laser beam monitor out of the treatment laser beam, if the one or more pre-defined tolerance limits are satisfied.

In an embodiment, the control module is configured to monitor, continuously during treatment, using the secondary signal, the secondary signal characteristics, and determine whether the secondary signal characteristics satisfy one or more pre-defined operating criteria. The control module is configured to stop the treatment laser beam from exiting the application head, if the one or more secondary signal characteristics do not satisfy the pre-defined operating criteria

In an embodiment, the control module is configured to monitor, continuously during treatment, using the secondary signal, the secondary signal characteristics. The secondary signal characteristics include a beam position. The control module is configured to record the secondary signal characteristics during treatment. The control module is configured to generate a dose map using the recorded secondary signal characteristics. The dose map is indicative of the energy distributed in the eye.

In an embodiment, the primary laser beam monitor comprises a photodiode and the primary signal characteristics include a beam power, a pulse energy, and/or a beam position. The secondary laser beam monitor comprises a photodetector array and the secondary signal characteristics include a beam power, a pulse energy, a beam position, a beam orientation, and/or a beam profile.

In an embodiment, the pre-defined operating criteria include an upper limit, a lower limit, and/or a range of one or more of the following: a beam power, a pulse energy, a beam position, a beam orientation, and/or a beam profile.

In an embodiment, the control module is configured to adjust the treatment laser beam, using the primary signal characteristics or the secondary signal characteristics, by controlling the treatment laser source, a laser attenuator, a beam shaper, and/or a scanner system.

In an embodiment, the secondary laser beam monitor is arranged behind a beam-splitter.

In an embodiment, the primary laser beam monitor comprises a sensor device of the following type(s): a photodiode, a photodetector array, a thermopile, a position sensitive device, an optical power sensor, a microbolometer, and/or a pyroelectric detector.

In an embodiment, the secondary laser beam monitor comprises a sensor device of the following type(s): a photodiode, a photodetector array, a thermopile, a position sensitive device, an optical power sensor, a microbolometer, and/or a pyroelectric detector.

In an embodiment, a sensor device of the primary laser beam monitor and a sensor device of the secondary laser beam monitor have a different physical sensing principle. Specifically, the primary laser beam monitor includes a first sensor device having a first physical sensing principle and the secondary laser beam monitor includes a second sensor device having a second physical sensing principle, the second physical sensing principle being different from the first physical sensing principle.

In an embodiment, the base station further comprises a pilot light source including a pilot laser source and/or a pilot light-emitting diode. The pilot light source is coupled into the beam path, and the secondary signal includes a pilot signal from the pilot light source.

In an embodiment, the secondary laser beam monitor is arranged behind a beam-splitter configured to partially reflect the treatment laser beam and partially transmit the light from the light signal source.

In an embodiment, the primary signal characteristics and the secondary signal characteristics relate to one or more of the following properties of the treatment laser beam: a beam position, a beam orientation, a beam power, a pulse energy, and/or a beam profile.

In an embodiment, the control module is further configured to generate an alarm message if at least one of the primary signal characteristics and/or at least one of the secondary signal characteristics do not satisfy the pre-defined tolerance limits.

In addition to an ophthalmological laser treatment device, the present disclosure also relates to a method for controlling an ophthalmological laser treatment device. The ophthalmological laser treatment device comprises a base station having a treatment laser source configured to generate a treatment laser beam, a control module, an application head. The ophthalmological laser treatment device comprises an arm arranged between the base station and the application head configured to provide a beam path for the treatment laser beam; wherein the application head includes a primary laser beam monitor which is retractable out of the treatment laser beam and a secondary laser beam monitor. The method comprises moving, by the control module, the primary laser beam monitor into the treatment laser beam. The method comprises receiving, in the control module, a primary signal from the primary laser beam monitor and a secondary signal from the secondary laser beam monitor. The method comprises determining, in the control module, using the primary signal, primary signal characteristics and determining, using the secondary signal, secondary signal characteristics. The method comprises determining, in the control module, whether one or more of the primary signal characteristics and one or more of the secondary signal characteristics satisfy one or more pre-defined tolerance limits. The method comprises retracting, by the control module, the primary laser beam monitor out of the treatment laser beam, if the one or more pre-defined tolerance limits are satisfied.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- Fig. 1: shows a block diagram illustrating schematically an ophthalmological laser treatment device having a laser beam monitor;
- Fig. 2: shows a perspective view of an ophthalmological laser treatment device with a horizontally rotatable arm;
- Fig. 3: shows a perspective view of an ophthalmological laser treatment device with a vertically rotatable arm;
- Fig. 4: shows a perspective view of an ophthalmological laser treatment device with an articulated arm;
- Fig. 5a: shows a diagram illustrating schematically an ophthalmological laser treatment device with the primary laser monitor moved into the treatment laser beam path;
- Fig. 5b: shows a diagram illustrating schematically an ophthalmological laser treatment device with the primary laser monitor retracted out of the treatment laser beam path;
- Fig. 6: shows a flow diagram illustrating a number of steps for beam monitoring of an ophthalmological laser treatment device;
- Fig. 7: shows a flow diagram illustrating a number of optional further steps for beam monitoring of an ophthalmological laser treatment device;
- Fig. 8: shows a flow diagram illustrating a number of optional further steps for generating a dose map; and
- Fig. 9: shows a flow diagram illustrating a number of optional further steps for adjusting the treatment laser beam.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

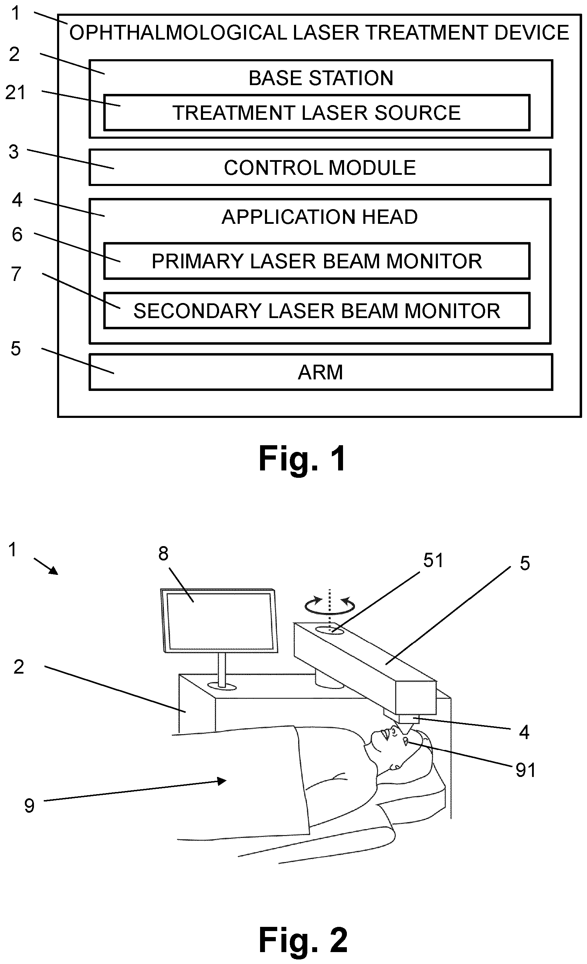

**Figure** 1 shows a block diagram illustrating schematically an ophthalmological laser treatment device 1. Figure 1 and also the remaining diagrams, in particular as shown in Figures 2-5b schematically illustrate modules and/or elements of various embodiments of the ophthalmological laser treatment device 1 and give an exemplary sequence or arrangement of modules and/or elements, including modules and/or elements in a beam path. The skilled person understands that at least some modules and/or elements shown in a particular Figure may be combined with modules and/or elements shown in another Figure. The ophthalmological laser treatment device 1 comprises a base station 2. The base station 2 is configured as a fixed or mobile apparatus. The ophthalmological laser treatment device 1 has a treatment laser source 21 which generates a treatment laser beam T.

The treatment laser source 21 is configured to, for example, generate an ultraviolet treatment laser beam T having a wavelength of between 190 nm and 230 nm. For example, the treatment laser source 21 comprises an excimer or a solid-state laser which produces such an ultraviolet treatment laser beam T. The excimer laser uses a combination of a noble gas and a reactive gas under high pressure and electrical stimulation to generate the treatment laser beam T. In particular, an excimer laser using argon as the noble gas and fluoride as the reaction gas may be used as the treatment laser source 21.

In another example, the treatment laser source 21 is configured to generate an infrared treatment laser beam T having a wavelength of between 780 nm and 1100 nm. For example, the treatment laser source 21 comprises a solid-state laser, such as a frequency converted Nd:YLF laser. The treatment laser source 21 will not be described in further detail, however the skilled person is aware that the treatment laser source 21 can comprise, for example, a gain medium, a laser resonator, a laser pump, a pulse generating element, cavity mirrors, coupling mirrors, wavelength tuners, and/or a frequency converter (including one or more non-linear optical crystals).

Depending on the embodiment, the treatment laser beam T is a continuous laser beam or a pulsed laser beam.

In an embodiment, the treatment laser source 21 is configured to generate femtosecond laser pulses, which have pulse widths of typically from 10 fs to 1000 fs (1 fs = 10⁻¹⁵ s).

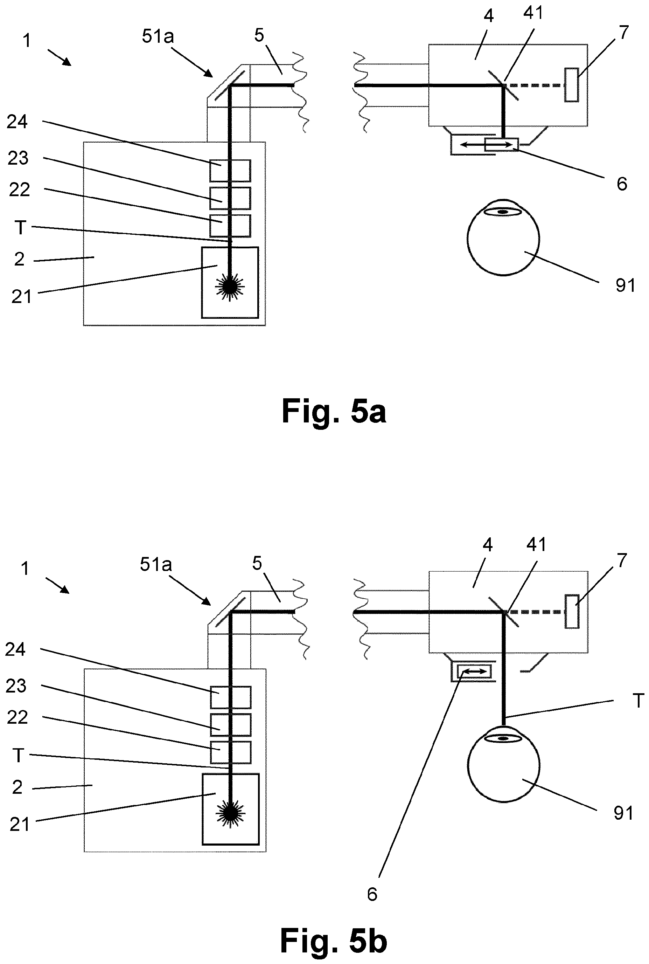

Downstream of the treatment laser source 21, the base station 2 includes an optional laser attenuator 22 (as shown in Figures 5a and 5b) configured to attenuate the treatment laser beam T.

Depending on the embodiment, a beam shaper 23 (as shown in Figures 5a and 5b) is included in the base station 2, arranged downstream from the treatment laser source 21. The beam shaper is configured to control the laser beam profile, in particular to redistribute the irradiance and/or phase of the treatment laser beam T to attain a desired laser beam profile that is maintained along the propagation distance, in particular the propagation distance from the beam shaper 23 to the eye 91 (as shown, for example, in Figures 5a and 5b).

The ophthalmological laser treatment device 1 further includes, in an embodiment, a shutter arranged in the beam path and configured to stop the treatment laser beam T if an appropriate shutter signal is received. The shutter is implemented, for example, in the base station 2, however it can also be implemented in the arm 4 or in the application head 3.

The base station 2 further includes a scanner system 24 (as shown in Figures 5a and 5b) which is configured to steer the treatment laser beam T delivered by the treatment laser source 21 onto treatment points on a treatment pattern (comprising a laser trajectory). In an embodiment, the scanner system 24 comprises a divergence modulator for modulating the focal depth, or the treatment height, in the projection direction along a projection axis. The scanner system 24 comprises, for example, a galvanoscanner comprising of one or more controllable mirrors. Alternatively, the scanner system 24 is arranged at least partly in the arm 4 and/or in the application head 3.

In an embodiment, the ophthalmological laser treatment device 1, in particular the base station 2, further includes a beam expander configured to alter a diameter of the treatment laser beam T.

The ophthalmological laser treatment device 1 comprises an application head 4. The application head 4 is designed to guide the treatment laser beam T into or onto the eye 91 of a patient 9 (as shown, for example, in Figures 2 to 4). The application head 4, for this purpose, can comprise focusing optics configured to focus the treatment laser beam T onto one or more treatment points inside or on the eye tissue, in particular the cornea for a pointwise tissue disruption or ablation. The focusing optics comprise a lens system having one or more optical lenses or mirrors. Depending on the embodiment, the focusing optics comprise one or more movable or deformable lenses and/or a drive for moving the entire focusing optics in order to set and adjust the focal depth, or the treatment height, in the projection direction along the projection axis. In a further embodiment, a divergence modulator is provided in the beam path between the treatment laser source 21 and the scanner system 24.

In an embodiment, the application head 4 comprises a patient interface. The application head 4 is preferably fixed onto the eye 91 by means of the patient interface, for example using suction.

Alternatively, the application head 4 does not have a patient interface for direct contact with the eye 91, but the application head 4 and the eye 91 of the patient 9 are separated by an air gap of several centimeters, for example.

Depending on the embodiment, the application head 4 further comprises an eye tracker configured to track a position and/or orientation of the eye 91 of the patient 9.

The ophthalmological laser treatment device 1 comprises an arm 5 arranged between the base station 2 and the application head 4. The arm 5 is configured to provide a beam path for the treatment laser beam T, such that the treatment laser beam T travels along the inside of the arm 5 from the base station 2 to the application head 4. In an embodiment, the arm 5 comprises one or more joints 51 (as shown in Figures 2-4) such that the application head 4 is movable and/or rotatable with respect to the base station 2. Each rotatable joint 51 comprises a mirror arranged in the beam path to reflect the treatment laser beam T along the arm 5.

Because the treatment laser beam T is reflected by each mirror, for example the mirrors in the arms 5, the treatment pattern generated by the scanner system 24 is reflected and/or rotated according to the angles between the beam path and the mirror. Additionally, the treatment laser beam T can shift laterally from its intended position due to imperfectly arranged mirrors, mechanical tolerances at the joints, thermal expansion of the arm 5, the joints 51, or other components of the ophthalmological laser treatment device 1, etc. As a result, the beam position and/or the beam orientation are not static, i.e. they change during operation of the ophthalmological laser treatment device 1. A change in the beam orientation may be apparent only once the scanner system 24 steers the treatment laser beam T according to an intermediate check or treatment model, in which case a pattern generated by the treatment laser beam T according to the intermediate check or treatment model will be rotated.

The treatment laser beam T, when exiting the application head 4, has properties which depend not only to the treatment laser source 21, but also on all intermediate components of the ophthalmological laser treatment device 1, including, but not limited to, the laser attenuator 22, the beam shaper 23, the scanner system 24, the arm 5 (including mirrors), and the application head 4. Monitoring these properties and ensuring that the treatment laser beam T satisfies one or more pre-defined tolerance limits and, optionally, pre-defined operating criteria, is paramount to ensuring safe and reliable treatment. The properties of the treatment laser beam T include a beam position or a beam orientation. These properties are influenced by the arm 5 and do not necessarily need to be determined using the treatment laser beam T, but can also be determined using a pilot light as is described below. Further properties of the treatment laser beam T include a beam power, a pulse energy, or a beam profile. These further properties are determined by monitoring the treatment laser beam T directly.

The ophthalmological laser treatment device 1 comprises a primary laser beam monitor 6 and a secondary laser beam monitor 7 for monitoring the treatment laser beam T. The primary laser beam monitor 6 is retractably arranged in the application head 4, such that in a first position, the primary laser beam monitor 6 intercepts the treatment laser beam T. As such, in the first position, the treatment laser beam T is incident on the primary laser beam monitor 6, and the primary laser beam monitor 6 measures properties (i.e. physical properties) of the treatment laser beam T. In a second position, the primary laser beam monitor 6 does not intercept the treatment laser beam T and allows the treatment laser beam T to pass unimpeded.

In an embodiment, the primary laser beam monitor 6, in the first position, does not allow the treatment laser beam T to pass (i.e. it stops the treatment laser beam T). In another embodiment, the primary laser beam monitor 6, in the first position, allows at least part of the treatment laser beam T to pass, e.g., by being partially transparent.

The primary laser beam monitor 6 is movable from the first position to the second position by an actuator connected to a control module 3, which control module 3 is described below in more detail. The actuator is configured to receive control signals from the control module 3 and to move the primary laser beam monitor 6 from the first position to the second position, and vice versa, upon receiving an appropriate control signal. Depending on the embodiment, the primary laser beam monitor 6 is continuously movable between the first position and the second position, or discretely movable.

The primary laser beam monitor 6 is arranged to be retractable by, for example, being laterally displaceable by the actuator. For example, the primary laser beam monitor 6 is displaceable in a horizontal direction, with respect to the application head 4 being positioned for treatment above a patient 9. In another example, the primary laser beam monitor 6 is arranged to be retractable by being rotatable about an axis by the actuator, such that in a first position the primary laser beam monitor 6 intercepts the treatment laser beam T and in a second position the primary laser beam monitor 6 is rotated out of the beam path.

The primary laser beam monitor 6 is configured to monitor the properties of the treatment laser beam T. Thereby, the primary laser beam monitor T also monitors the beam path inside the arm 5 along which the treatment laser beam T travels.

The primary laser beam monitor 6 is preferably arranged downstream of the last optical element of the ophthalmological laser treatment device 1, in particular downstream of the focusing optics, such that the primary beam monitor 6 measures the treatment laser beam T as it would strike the eye tissue of the eye 91. This establishes the primary signal of the primary beam monitor 6 as a ground truth of the properties of the treatment laser beam T, in particular for comparison with the secondary signal of the secondary beam monitor 6.

In an embodiment, the primary laser beam monitor 6 is integrally mounted in the application head 4. For example, primary laser beam monitor 6 is mounted in the application head 4 such that the primary laser beam monitor 6 is substantially enclosed by the application head 4. In another example, the primary laser beam monitor 6 is arranged such that at least part of a housing of the primary laser beam monitor 6 has a shape complementary to a cutout or recess of the application head 4. In another example, the primary laser beam monitor 6 is arranged such that at least part of the housing of the primary laser beam monitor 6 is substantially flush with a housing of the application head 4. The examples described do not place the primary laser beam monitor 6 in a treatment plane, i.e. in a position where the eye 91 of the patient 9 is during treatment, as such an arrangement would not be integrally mounted. Rather, the primary laser beam monitor 6 is arranged to intercept the treatment laser beam upstream of the treatment plane. The integral arrangement ensures a consistent measurement is easily achieved, without having to manually attach (and later, remove) the primary laser beam monitor 6 to the application head 4.

In an example, the application head 4 fully encloses the primary laser beam monitor 6 when the primary laser beam monitor 6 is in the first position and when the primary laser beam monitor 6 is in the second position.

In an example, the application head 4 has an opening through which the treatment laser beam travels out of the application head 4. The primary laser beam monitor 6 is configured to substantially cover, preferably fully cover, the opening when in the first position and to at least partially uncover, preferably fully uncover, the opening when in the second position.

The examples described above do not place the primary laser beam monitor 6 in the treatment plane, thereby allowing for the primary laser beam monitor to monitor the treatment laser beam directly prior to treatment, in particular with the patient 9 positioned for treatment with the eye 91 of the patient 9 in the treatment plane. Further, the examples ensure that the outer dimensions of the application head 4 remain substantially unchanged regardless of whether the primary laser beam monitor 6 is in the first position or the second position, which increases ease of use when positioning the application head 4.

In an embodiment, the primary laser beam monitor 6 is configured such that, when the primary laser beam monitor 6 is in the first position, the treatment laser beam T is directly incident on the primary laser beam monitor 6. In other words, the treatment laser beam T does not pass through any pattern, mask, or any other means arranged directly upstream from the primary laser beam monitor 6 which may partially obscure, alter, or otherwise influence the treatment laser beam T.

Additionally to monitoring the treatment laser beam T, the primary laser beam monitor 6 can further be configured to monitor a pilot light which has traveled through the arm 5 along the beam path. The pilot light originates from a pilot light source in the base station 2 where it is coupled into the same beam path as the treatment laser beam T. The pilot light source is described below in more detail. As the pilot light is also affected (e.g. reflected, attenuated, rotated, and/or laterally shifted) by the same mirrors or other optical components arranged in the beam path inside the arm 5, by monitoring the pilot light the ophthalmological laser treatment device 1 is capable of determining how the treatment laser beam T is also affected by the same mirrors and/or other optical components.

Depending on the embodiment, the primary laser beam monitor 6 comprises a photodetector array, for example a one-dimensional, two-dimensional, or three-dimensional array of photodetectors. The photodetector array is implemented, depending on the embodiment, as a CCD sensor or a CMOS sensor, for example. The photodetector array may comprise a movable shutter.

The photodetector array may be configured to determine one or more properties of the treatment laser beam T, in particular a beam power, a pulse energy, and/or a beam profile. In particular, the photodetector array may be configured to measure the one or more properties simultaneously. The photodetector array may be configured such that, when the primary laser beam monitor 6, for example, is in the first position, the entire treatment laser beam T, in particular a full width of the treatment laser beam T, is entirely incident on the photodetector array. Further, the photodetector array may be configured to measure the properties instantaneously, i.e. at a given and well-defined time-point or within a defined small time-window (e.g. preferably in less than 100 milliseconds, more preferably in less than 10 milliseconds). The photodetector array may be configured to measure the properties without intermediary means, in particular without intermediary means such as patterns, masks, and/or filters arranged immediately upstream of the photodetector array, in particular without moveable intermediary means.Additionally or alternatively, the primary laser beam monitor 6 comprises a photodiode, a thermopile, a position sensitive device, an optical power sensor, a microbolometer, and/or a pyroelectric detector. The aforementioned sensors can be arranged on a movable track which track may additionally be rotatable. Thereby, the aforementioned sensors are also capable of measuring a beam position and/or a beam orientation.

The photodetector array is configured, depending on the embodiment, to be sensitive to one or more wavelengths (this includes a wavelength band) of light. The wavelengths to which the photodetector array may be sensitive are not limited to visible wavelengths, but are additionally or alternatively also sensitive to light beyond the visible range, for example ultraviolet light and/or infrared light.

The primary laser beam monitor 6 is configured to generate a primary signal (e.g. in the form of a digital signal) indicative of the measured properties of the treatment laser beam and/or the pilot light. The primary laser beam monitor 6 is connected to the control module 3 and the control module 3 is configured to receive the primary signal from the primary laser beam monitor 6. The primary signal is, for example, a digital signal.

In an embodiment, the primary laser beam monitor 6 further comprises one or more filters. The filters are configured to absorb, scatter, convert and/or reflect one or more wavelengths of light. Depending on the embodiment, the filters may further be configured to re-emit energy any absorbed energy in a specific wavelength region.

The filters comprise, for example, one or more color filters, one or more spectral filters, one or more neutral density filters, one or more bandpass filters, one or more notch filters, one or more edge filters, one or more beamsplitter filters, one or more dichroic filters, one or more color substrate filters, one or more excitation filters, and/or one or more emission filters. The filters can cover the entire primary laser beam monitor 6 or parts thereof, including, for example, individual areas (e.g., a Bayer color filter may be used where the primary laser beam monitor 6 is implemented as a photodetector array).

The primary laser beam monitor 6 further comprises, in an embodiment, optical elements such as mirrors and/or lenses to focus and/or distribute the light signal across the photodetector array. For example, the optical elements include microlenses arranged in front of the photodetector array to increase the light signal detected at each photodetector.

The ophthalmological laser treatment device 1 comprises a secondary laser beam monitor 7. The secondary laser beam monitor 7 is configured to monitor the treatment laser beam T, preferably continuously. The secondary laser beam monitor 7 is arranged in the application head 4. For monitoring the treatment laser beam T, secondary laser beam monitor 7 intercepts at least part of the treatment laser beam T. As such, the secondary laser beam monitor 7 intercepts the treatment laser beam T, or part of it, and the secondary laser beam monitor 7 measures properties (i.e. physical properties) of the treatment laser beam T. The secondary laser beam monitor 7 is configured to generate a secondary signal (e.g., in the form of a digital signal) which is indicative of the measured properties of the treatment laser beam T and/or the pilot light. Specifically, the secondary signal indicates current measured properties of the treatment laser beam T and/or the pilot light.

The secondary laser beam monitor 7 is, for example, arranged behind a beam-splitter 41 (as shown in Figures 5a and 5b), the beam-splitter 41 being configured to divert part of the treatment laser beam 41 onto the secondary laser beam monitor 7. In another example, the secondary laser beam monitor 7 is partially transparent and arranged in the beam path of the treatment laser beam T. The beam splitter 41 is, for example, implemented using a partially transparent mirror.

The beam splitter 41, behind which the secondary laser beam monitor 7 is arranged, is preferably arranged immediately upstream to the primary laser beam monitor 6. In other words, when the primary laser beam monitor 6 is in the first position, the part of the treatment laser beam T which, after interacting with the beam splitter, does not fall onto the secondary laser beam monitor 7 is directly incident onto the primary laser beam monitor 6, in particular with no intermediary components which interact with the treatment laser beam T.

Depending on the embodiment, the secondary laser beam monitor 7 comprises a photodetector array, for example a one-dimensional, two-dimensional, or three-dimensional array of photodetectors. The photodetector array is implemented, depending on the embodiment, as a CCD sensor or a CMOS sensor, for example.

Additionally or alternatively, the secondary laser beam monitor 7 comprises a photodetector, a thermopile, a position sensitive device, an optical power sensor, a microbolometer, and/or a pyroelectric detector. The aforementioned sensors can be arranged on a movable track which track may additionally be rotatable. Thereby, the aforementioned sensors are also capable of measuring a beam position and/or a beam orientation.

The above-described details and embodiments of the primary laser beam monitor 6 also apply to the secondary laser beam monitor 7. In particular, the details regarding the photodetector array, filters, and optical elements described with reference to the primary laser beam monitor 6 are also, depending on the embodiment, implemented in the secondary laser beam monitor 7.

The secondary laser beam monitor 7 is therefore configured, much like the primary laser beam monitor 6, to monitor properties of the treatment laser beam T and monitor the beam path. The ophthalmological laser treatment device 1 is thereby configured to redundantly monitor the treatment laser beam T which increases the safety of operation of the ophthalmological laser treatment device 1.

In an embodiment, the primary laser beam monitor 6 and the secondary laser beam monitor 7 employ the same physical sensing principle. Preferably, the primary signal characteristics and the secondary signal characteristics relate to the same one or more properties of the treatment laser beam T. Preferably, the primary laser beam monitor 6 and the secondary laser beam monitor 7 are implemented using two identical sensors.

In an embodiment, the primary laser beam monitor 6 and the secondary laser beam monitor 7 employ a different physical sensing principal and therefore implement a different type of sensor. Thereby, a redundant intermediate check of the ophthalmological laser treatment device 1 is made possible which eliminates at least some systematic measurement errors related to particular types of sensor.

In an example, the primary laser beam monitor 6 comprises a microbolometer and the secondary laser beam monitor 7 comprises a photodetector array.

In an example, the primary laser beam monitor 6 comprises a photodiode. The primary signal characteristics determined by the control module 3 include a beam power and/or a pulse energy. The secondary laser beam monitor 7 comprises a photodetector array. The secondary signal characteristics determined by the control module 3 include a beam position, a beam profile and/or a beam power. The control module 3 is optionally further configured to determine a beam orientation. In this example, the beam power is redundantly measured as both the primary laser beam monitor 6 and the secondary laser beam monitor 7 provide a measurement signal allowing the control module 3 to determine the beam power as measured by both laser beam monitors 6, 7 separately. The further mentioned properties of the treatment laser beam T are, in this example, determined by the secondary laser beam monitor 7.

The ophthalmological laser treatment device 1 comprises, in an embodiment, a pilot light source configured to generate the pilot light which is detected by the primary laser beam monitor 6 and/or the secondary laser beam monitor 7. The pilot light source is arranged, for example, in the base station 2.

The ophthalmological laser treatment device 1, in particular using the control module 3 described below, determines properties of the treatment laser beam T by monitoring the treatment laser beam T directly using the primary laser beam monitor 6 and/or the secondary laser beam monitor 7. Alternatively or additionally, the ophthalmological laser treatment device 1 monitors the treatment laser beam T indirectly by monitoring the pilot light, the pilot light being monitored using the primary laser beam monitor 6 and/or the secondary laser beam monitor 7. This is possible because the properties of both the pilot light, as produced by the pilot light source, as well as the treatment laser source 21 are known and because both the treatment laser beam T as well as the pilot light travel down the arm 3 along same beam path, whose properties (e.g. due to the mirrors and/or other optical components in the arm 3) are known. In particular, the control module 3 receives, from the primary laser beam monitor 6 and/or the secondary laser beam monitor 7 the primary signal and/or the secondary signal, respectively, and determines the properties of the treatment laser beam T using the primary signal and/or the secondary signal.

Additionally, or alternatively, the control module 3 is configured to monitor the scanner system 24 using the properties of the monitored treatment laser beam T and/or the pilot light, in particular a beam position and/or beam orientation of the treatment laser beam T. Monitoring the scanner system 24 comprises, for example, calibrating the scanner system 24 and/or testing the scanner system 24. In an embodiment, the pilot light source is a laser source which is separate to the treatment laser source 21. The pilot laser source has known characteristics (e.g., wavelength, pulse length) which are, depending on the embodiment, different than the treatment laser source 21.

In an embodiment, the pilot light source includes a pilot light-emitting diode. The light-emitting diode (LED) can be implemented as a single LED or an array of LEDs. The one or more LEDs can be configured to produce one or more wavelengths of light.

The ophthalmological laser treatment device 1 comprises a control module 3 configured to control the ophthalmological laser treatment device 1. The control module 3 is preferably arranged in the base station 2. The control module 3 embodies a programmable device and comprises, for example, one or more processors, and one or more memory modules having stored thereon program code, data, as well as programmed software modules for controlling the processors, and/or other programmable circuits or logic units included in the control module 3, such as ASICs (Application-Specific Integrated Circuits), GPUs (graphics processing units), and/or TPUs (tensor processing units). The memory modules comprise volatile and/or non-volatile storage media, for example random access memory and/or flash memory, respectively. The control module 3 is connected to other components and modules of the ophthalmological laser treatment device 1 as disclosed herein, in particular the treatment laser source 21, the laser attenuator 22, the beam shaper 23 the scanner system 24, the primary laser beam monitor 6, the secondary laser beam monitor 7, and optionally the pilot light source. The connection is a wired and/or wireless connection configured to exchange control and/or measurement signals.

The control module 3, depending on the embodiment, further comprise a communication interface. The communication interface is configured for data communication with one or more external devices. Preferably, the communication interface comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The control module 3 performs one or more steps and/or functions as described herein, for example according to the program code stored in the one or more memory modules. Additionally, or alternatively, the program code can be wholly or partially stored in one or more auxiliary processing devices, for example a computer. The skilled person is aware that at least some of the steps and/or functions described herein as being performed on the processor of the ophthalmological laser treatment device 1 may be performed on one or more auxiliary processing devices connected to the ophthalmological laser treatment device 1 using the communication interface. The auxiliary processing devices can be colocated with the ophthalmological laser treatment device 1 or located remotely, for example on a remote server computer.

The skilled person is also aware that least some of the data associated with the program code (application data) or data associated with a particular patient (patient data) and described as being stored in the memory of the ophthalmological laser treatment device 1 may be stored on one or more auxiliary storage devices connected to the ophthalmological laser treatment device 1 using the communication interface.

The control module 3 stores, in the one or more memory modules, a treatment model. The treatment model is designed for treating the eye 91 of a patient 9 using the ophthalmological laser treatment device 1. In particular, the treatment model defines a number of treatment points or treatment curves onto which the treatment laser beam T is directed.

The control module 3 is configured to store, in the one or more memory modules, intermediate check data. The intermediate check data is generated during an intermediate check. To this end, the one or more memory modules further comprise, for example, program code configured such that the control module 3 performs the intermediate check. The intermediate checks are performed in smaller increments of time than the calibration, for example prior to each treatment, such that it is ensured that the ophthalmological treatment device 1 is performing according to specification, in particular that the primary signal characteristics and the secondary signal characteristics, as determined using the primary signal and the secondary signal, respectively, satisfy one or more pre-defined tolerance limits. The intermediate check may involve various components of the ophthalmological laser treatment device 1, for example the treatment laser source 21, the pilot light source, and/or the scanner system 24. The intermediate check takes place with the primary laser beam monitor 6 in the first position. In an embodiment where the ophthalmological laser treatment device 1 comprises a shutter, the intermediate check can also be performed with the patient 9 in position under the application head 91.

The one or more pre-defined tolerance limits mentioned herein are established during calibration of the ophthalmological laser treatment device 1, which takes place prior to the intermediate check, for example during commissioning of the ophthalmological laser treatment device 1 or during regular scheduled calibration (which takes place less frequently than the intermediate check, for example, once daily).

During calibration, the treatment laser beam T is directed onto a reference surface (made of polymethyl methacrylate (PMMA), for example) mounted in the treatment plane, i.e. at a treatment distance from the application head 4, in particular where the eye 91 of the patient 9 will be located during treatment. The reference surface is ablated for a pre-determined period of time or a pre-determined number of laser pulses while the treatment laser source is set to a pre-determined treatment laser power. The reference surface is then measured, for example using interferometry, and a relation is established between the pre-determined treatment laser power (typically expressed in milliwatts) and the rate of ablation (typically expressed in micrometers per second). The established relation is checked against particular calibration limits, such that if the pre-determined treatment laser power does not lead to a rate of ablation within the particular calibration limits, the ophthalmological treatment laser device 1 is not cleared for use during treatment. The calibration may additionally include ablating and/or cutting according to a pre-defined grid pattern, allowing for simple visual identification of positional deviations.

After calibrating the ophthalmological laser treatment device 1 using the reference surface, the primary laser beam monitor 6 and the secondary laser beam monitor 7 are both exposed to the treatment laser beam T, preferably at the same power as during the calibration. Thereby, the primary signal and the secondary signal are "zeroed". The pre-defined tolerance limits define a permissible deviation of the primary signal and the secondary signal to the "zeroed" values, for example 1%, 2%, or 5%. After both calibration and zeroing, the ophthalmological treatment device 1 is considered approved for treatment for a particular duration of time (for example, for a given day or week), or a particular number of treatments (for example 10 treatments or 50 treatments). The intermediate check is then executed, for example prior to each treatment, or at a regular time-interval (e.g. every 30 minutes, every 60 minutes, or every 120 minutes) to check whether the ophthalmological treatment laser device 1 is still operating within the pre-defined tolerance limits. As the intermediate check takes less time than the calibration, the intermediate check saves time while maintaining safe operation of the ophthalmological treatment laser device 1. Additionally, as the intermediate check is carried out fully automatically, there is no manual work involved.

In an embodiment, the control module 3 is configured to store, in the one or more memory modules, pre-defined operating criteria. The control module 3 is configured to continuously monitor, during treatment, whether the ophthalmological treatment device 1 is operating within the pre-defined operating criteria, in particular whether the secondary signal characteristics, as determined by the control module 3 using the secondary signal, satisfy one or more of the pre-defined operating criteria. The pre-defined operating criteria may be identical with the pre-defined tolerance limits used for the intermediate check. The pre-defined operating criteria may also be defined relative to the results of the intermediate check.

The ophthalmological laser treatment device 1 optionally includes a user interface comprising, for example, one or more user input devices, such as a keyboard, and one or more output devices, such as a display 8. The user interface is configured to receive user inputs from an eye treatment professional, in particular based on, or in response to, information displayed to the eye treatment professional using the one or more output devices.

**Figures 2** to **4** show three different embodiments of the ophthalmological laser treatment device 1, each having a different embodiment of the arm 5. Other embodiments are possible, and depending on the configuration of the base station 2, in particular whether the base station 2 is itself height-adjustable or not, certain aspects of at least some of the three embodiments described below can be modified. Specifically, certain joints 51 for adjusting a vertical height of the application head 4 are superfluous, depending on the embodiment, and may be omitted.

In **Figure 2****,** the arm is rotatable about the joint 51, such that the arm 5 can swing horizontally over a reclining patient 9 such that the application head 4 is moved into position above the eye 91 of the patient 9.

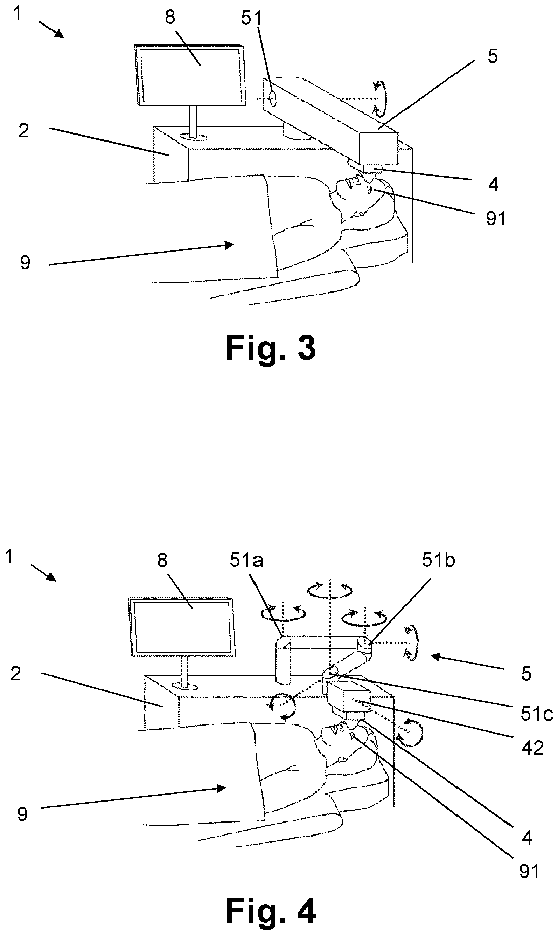

In **Figure 3****,** the arm 5 is rotatable about the joint 51 such that the arm 5 can swing vertically over a reclining patient 9 such that the application head 4 is moved into position above the eye 91 of the patient 9.

In **Figure 4****,** the arm 5 is an articulated arm 5 rotatable about the joints 51a, 51b, 51c such that the application head 4 can be flexibly moved into position above the eye 91 of the patient 9. Each joint 51a, 51b, 51c enables one or more rotations, for example the joint 51b allows both a rotation in the vertical as well as the horizontal plane. Additionally, it can be seen that the application head 4 has a joint 42 about which the application head 4 can be rotated.

**Figures 5a** and **5b** show schematic block diagrams of different embodiments of the ophthalmological laser treatment device 1, in particular showing the primary laser beam monitor 6 in the first position and the second position, respectively. Figures 5a and 5b both show the ophthalmological laser treatment device 1 as having a rotatable arm 4 which can swing horizontally over a reclining patient 9, as is also shown in Figure 2, however this is for purposes of illustration only and is not intended to be limiting. Specifically, the particular arrangements of the primary laser beam monitor 6 and the secondary laser beam monitor 6 as shown are transferable to other embodiments of the present disclosure in which the arm 5 is fixed, rotatable, and/or telescopic, for example.

In **Figure 5a****,** the treatment laser source 21 is arranged in the base station 2. The treatment laser beam T generated by the treatment laser source 21 passes along a beam path from the treatment laser source 21 to the primary laser beam monitor 6, which is shown in the first position. The laser attenuator 22, the beam shaper 23, and the scanner system 24 are shown arranged in the beam path. The treatment laser beam T is reflected in the arm 5 by a mirror at the joint 51a and subsequently enters the application head 4. A cut-away section is shown in which further joints 51 may be arranged, each with a further mirror. In the application head, the treatment laser beam T is partially reflected towards the primary laser beam monitor 6 by the beam-splitter 41. A remaining portion of the treatment laser beam T is transmitted through the beam-splitter where it is incident on the secondary laser beam monitor 7.

With the primary laser beam monitor 6 in the first position as shown, the treatment laser beam T is blocked and does not enter the patient's eye 91. The treatment laser beam T is monitored by both the primary laser beam monitor 6 and the secondary laser beam monitor 7 simultaneously, thereby allowing for cross-checking of the primary signal and the secondary signal during the intermediate check. Specifically, the control module determines, using the primary signal and the secondary signal, primary signal characteristics and secondary signal characteristics, respectively, and checks whether they satisfy the one or more pre-defined tolerance limits.

Depending on the embodiment, the primary laser beam monitor 6 is regarded as measuring a ground truth (as indicated by the primary signal characteristics) and the control module 3 checks whether the secondary laser beam monitor 7 provides a measurement (in the form of the secondary signal characteristics) which is in agreement (i.e. whether they satisfy one or more pre-defined tolerance limits) with the ground truth. In this case, the pre-defined tolerance limits are defined as a deviation between the primary signal characteristics and the secondary signal characteristics.

In another embodiment, the control module 3 checks whether the primary signal characteristics and the secondary signal characteristics are in agreement with pre-determined laser setpoint characteristics which define one or more desired properties of the treatment laser beam T, in particular properties that the treatment laser beam T is to have upon exiting the application head 4. These properties include, for example, the beam power, the beam position, and/or the beam orientation. In this case, the pre-defined tolerance limits are defined as a deviation between the primary signal characteristics and the pre-determined laser setpoint characteristics, and as a deviation between the secondary signal characteristics and the pre-determined laser setpoint characteristics. Depending on the implementation, the intermediate check is considered passed only if both the primary signal characteristics and the secondary signal characteristics satisfy the pre-defined tolerance limits.

By performing the intermediate check, it is ensured that the treatment laser beam T is operating according to specification in a redundant manner. In particular, any changes in the beam path, in particular due to a rotation at the joints 51a, 51b, 51c and/or a thermal expansion or contraction which would change the properties of the treatment laser beam T are detected and it can be ensured that the treatment will take place in a safe manner.

**Figure 5b** differs from Figure 5a in that the primary laser beam monitor 6 is shown in the second position such that it is retracted laterally out of the beam path of the treatment laser beam T, using the actuator (not shown), such that the treatment laser beam T is incident on the patient's eye 91. During treatment, therefore, the primary laser beam monitor 6 does not monitor the treatment laser beam T. During treatment, the secondary laser beam monitor 7, arranged behind the beam-splitter 41 monitors the treatment laser beam T (preferably continuously), generating the secondary signal and transmitting it to the control module 3. The control module 3 monitors (preferably continuously) the secondary signal characteristics using the secondary signal and (preferably continuously) determines whether the secondary signal characteristics satisfy one or more pre-defined operating criteria. Thereby, safe operation of the ophthalmological laser treatment device 1 is ensured.

As above during the intermediate check, the continuous monitoring ensures that any changes in the beam path, for example due to a thermal expansion or contraction, and/or movement play due to mechanical tolerances, which would change the properties of the treatment laser beam T, is detected by the control module 3. Further, any variances in the treatment laser beam T due to changes in the treatment laser source 21 or any other intermediary components arranged in the beam path are also detected.

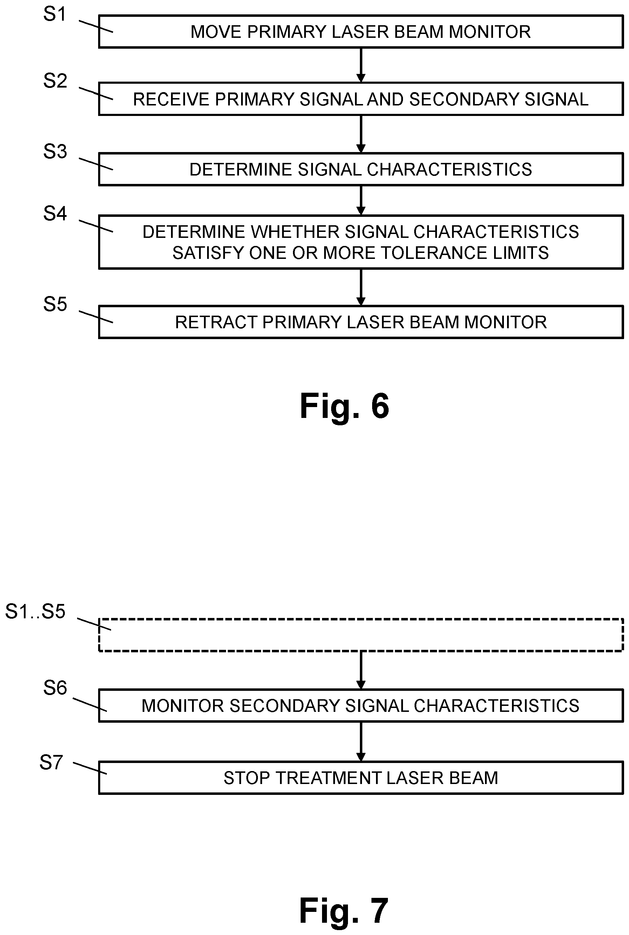

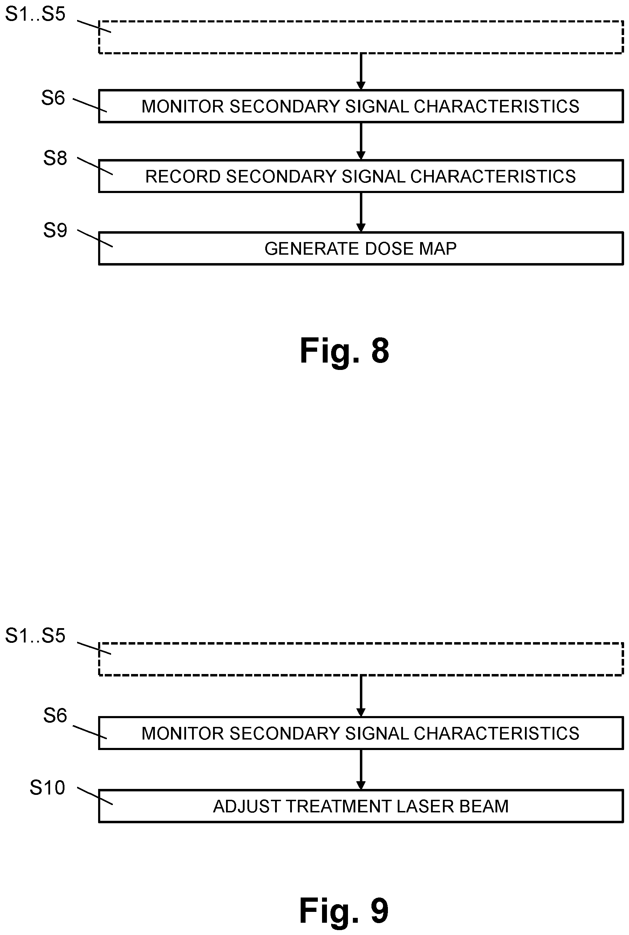

**Figures 6 to 9** show a flow diagrams illustrating a number of steps for operating the ophthalmological laser treatment device 1. **Figure 6** show steps S1 to S5, whilst **Figure 7** shows optional additional steps S6 and S7, **Figure 8** shows optional additional steps S6, S8, and S9, and **Figure 9** shows optional additional steps S6 and S10.

In a step S1, the control module 3 is configured to move the primary laser beam monitor 6 into the first position, in particular for the intermediate check. Specifically, the control module 3 is configured to send a control signal to the actuator such that the primary laser beam monitor moves into the first position.

In a step S2, the treatment laser source 21 and/or the pilot light source are powered on such that the treatment laser beam T and/or the pilot light are monitored by the primary laser beam monitor 6 and the secondary laser beam monitor 7 simultaneously. Thereby, properties of the treatment laser beam T are measured by the primary laser beam monitor 6 and the secondary laser beam monitor 7 at the same time. The measurement signals, i.e. the primary signal and the secondary signal, are generated by the primary laser beam monitor 6 and the secondary laser beam monitor 7, respectively, and transmitted to the control module 3, which receives the primary signal and the secondary signal.

In a step S3, the control module 3 determines primary signal characteristics using the received primary signal, and secondary signal characteristics using the received secondary signals.

In a step S4, the control module 3 determines whether the treatment laser beam T satisfies one or more pre-defined tolerance limits. In particular, the control module 3 determines whether one or more of the primary signal characteristics, which are associated with one or more properties of the treatment laser beam T, respectively, satisfy one or more of the pre-defined tolerance limits. The control module 3 also determines whether one or more of the secondary signal characteristics, which are associated with one or more properties of the treatment laser beam T, respectively, satisfy one or more of the pre-defined tolerance limits.

Depending on the embodiment, the primary signal characteristics and secondary signal characteristics relate to an overlapping or distinct set of properties of the treatment laser beam T.

In an embodiment, steps S2 to S4 occur simultaneously during the intermediate check. The treatment laser source 21 and/or the pilot light source, and optionally other components, for example the scanner system 24, are controlled, during this intermediate check, according to intermediate check control parameters. These intermediate check control parameters, for example, define the beam power and/or set the scanner system 24 to one or more pre-determined positions, for example a zero position (in the zero position, the scanner system 24 does not steer the treatment laser beam T off a center axis of the beam path). Additionally or alternatively, the intermediate check control parameters may also cause the scanner system 24 to generate a test pattern, such that the control module 3 is able to monitor the beam position and/or the beam orientation of the treatment laser beam T and ensure that the ophthalmological laser treatment device 1 is operating within the pre-defined tolerance limits.

In an embodiment, the control module 3 is configured to control the ophthalmological laser treatment device 1 by controlling the scanner system 24 using the signal characteristics. For example, the control module 3 is configured to transmit, to the scanner system 24, a control adjustment signal configured to control the scanner to laterally shift and/or rotate the treatment model, particularly during treatment, according to the primary signal characteristics and/or the secondary signal characteristics. Thereby, the planned treatment of the eye 91 of the patient 9 is unaffected by changes in the arm 5, for example due to thermal expansion or mechanical tolerances in the joints 51, because the ophthalmological laser treatment device 1 adjusts the treatment laser beam T to compensate for any such changes.

In an embodiment, the control module 3 is configured to control the ophthalmological laser treatment device 1 by controlling the treatment laser source 21 using the primary signal characteristics and/or the secondary signal characteristics.

In an embodiment, the control module 3 is configured to control the ophthalmological laser treatment device 1 by controlling the attenuator 22.

In an embodiment, the control is configured to control the ophthalmological laser treatment device 1 by controlling the shutter.

In an embodiment, the control module 3 is configured to determine, using the primary signal characteristics and/or the secondary signal characteristics, properties of the treatment laser beam T and is configured to control the treatment laser source 21 by determining these properties and adjusting the treatment laser source 21 accordingly. The properties include a beam position of the treatment laser beam T after exiting the arm, a rotational orientation of the treatment laser beam T after exiting the arm, a beam power of the treatment laser beam T, a laser pulse energy of the treatment laser beam T, and/or a laser beam profile of the treatment laser beam T. The properties of the treatment laser beam T can further include, for example, a beam dispersion, a beam central wavelength, and/or a beam wavelength distribution. The control module 3 is configured to control the ophthalmological laser treatment device 1 using one or more of the properties of the treatment laser beam T. For example, the control module 3 is configured to control the ophthalmological laser treatment device 1 such that the aforementioned properties satisfy the one or more pre-defined tolerance limits, e.g., have a particular set-point value or lie within a particular range around the set-point value.

In an embodiment, the control module 3 is further configured to generate an alarm message if the primary signal characteristics and/or the secondary signal characteristics do not satisfy one or more pre-defined tolerance limits (e.g. the primary signal characteristics and/or the secondary signal characteristics are above or below a maximum or minimum signal threshold, respectively, or the primary signal characteristics and/or the secondary signal characteristics are not within a range indicated by an upper and a lower signal threshold). The primary signal characteristics and/or the secondary signal characteristics define, for example, a maximum value and/or a minimum value of the beam power and/or of the laser pulse power. The alarm message can be, for example, prominently displayed on the display 8 of the ophthalmological laser treatment device 1. The ophthalmological laser treatment device 1 can further be configured to engage the shutter to stop the treatment laser beam T and/or power down the treatment laser source 21, in conjunction with the alarm message.

In a step S5, the control module 3 is configured to retract the primary laser beam monitor 6 out of the treatment laser beam T, if the one or more pre-defined tolerance limits are satisfied.

As explained above, the control module 3, if the pre-defined tolerance limits are not satisfied, for example shuts down the ophthalmological laser treatment device 1, in particular the treatment laser source 21, or, in another example, adjusts the ophthalmological laser treatment device 1, in particular the treatment laser source 21 or other components along the beam path, such that the pre-defined tolerance limits are satisfied.

After the primary laser beam monitor 6 has been retracted out of the beam path of the treatment laser beam 6, treatment of the patient's eye 91 takes place according to the treatment model. In particular, control module 3 is configured to control the ophthalmological laser treatment device 1 according to the treatment model.

As shown in **Figures 7** to **9****,** in a step S6, the control module 3 is configured to continuously monitor during treatment the secondary signal characteristics, using the continuously received secondary signal. The control module 3 is configured to determine whether the secondary signal characteristics satisfy one or more pre-defined operating criteria. The pre-defined operating criteria relate to one or more properties of the treatment laser beam T. The pre-defined operating criteria may correspond, at least in part, with the pre-defined tolerance limits described above.

The pre-defined operating criteria, depending on the embodiment, further relate to the treatment model, such that the control module 3 continuously monitors whether the treatment laser beam T is behaving according to pre-defined operating criteria specific to the treatment model.

In an embodiment, failing to satisfy the pre-defined operating criteria of the one or more secondary signal characteristics triggers a manual check of the treatment laser beam 1 that needs to be performed by the user. This manual check includes, for example, the calibration as described herein.

In **Figure 7****,** steps S1 to S6 relate to steps S1 to S6 as described above with reference In a further step S7, the control module 3 is configured to stop the treatment laser beam T from exiting the application head 4, if the one or more secondary signal characteristics do not satisfy the pre-defined operating criteria. For example, the control module 3 shuts off the treatment laser source 21 and/or closes the shutter. Alternatively or additionally, the primary laser beam monitor 6 is moved into the first position such that it blocks the treatment laser beam T.

In **Figure 8****,** in a further step S8, the control module 3 is configured to record the secondary signal characteristics, preferably to the memory module. The recorded secondary signal characteristics are used to generate, in a step S9, a dose map using the secondary signal characteristics. The dose map is a two-dimensional, quasi-two-dimensional (e.g. a curved plane), or three-dimensional map indicating an energy dose for each position of the eye 91 of the patient 9 which has been targeted by the treatment laser beam T.

For example, the dose map is generated by integrating, over time, the recorded secondary signal characteristics, in particular using the recorded secondary signal characteristics relating to the beam power and/or the pulse energy, and the beam position. Preferably, the dose map is continuously generated, respectively updated, during treatment by appending the dose map using currently determined secondary signal characteristics. Preferably, the dose map is provided to the operator of the ophthalmological laser treatment device 1. For example, the dose map is displayed on the display 8. Preferably, the dose map is continuously generated, respectively updated, such that the operator sees which parts of the eye 91 have received which amount of energy. Optionally, the dose map is generated by the control module 3 to include the treatment model, in particular the points of the treatment model not yet targeted by the treatment laser beam T.

In **Figure 9**, in an optional step S10, the control module 3 is configured to adjust the treatment laser beam T, if the one or more secondary signal characteristics do not satisfy the pre-defined operating criteria. Specifically, the treatment laser beam T is adjusted by controlling properties of the treatment laser beam T, in particular the beam position, the beam orientation, the beam power, the pulse energy, or the beam profile. The control module 3 controls the properties by sending appropriate control signals to the treatment laser source 21 and/or one or more other components along the beam path. These other components include, but are not limited to, the attenuator 22, the beam shaper 23, and the scanner system 24. The control module 3 is configured to adjust the treatment laser beam T, by controlling the properties until the treatment laser beam T satisfies the pre-defined operating criteria.

The above-described embodiments of the disclosure are exemplary and the person skilled in the art knows that at least some of the components and/or steps described in the embodiments above may be rearranged, omitted, or introduced into other embodiments without deviating from the scope of the present disclosure.

## Claims

1. An ophthalmological laser treatment device (1), the ophthalmological laser treatment device (1) comprising:
a base station (2) having a treatment laser source (21) configured to generate a treatment laser beam (T),
a control module (3),
an application head (4), and
an arm (5) arranged between the base station (2) and the application head (4) configured to provide a beam path for the treatment laser beam (T); wherein
the application head (4) includes a primary laser beam monitor (6) which is retractable out of the treatment laser beam (T) and a secondary laser beam monitor (7), and wherein the control module (3) is configured to:
move (S1) the primary laser beam monitor (6) into the treatment laser beam (T),
receive (S2) a primary signal from the primary laser beam monitor (6) and a secondary signal from the secondary laser beam monitor (7),
determine (S3), using the primary signal, primary signal characteristics and determine, using the secondary signal, secondary signal characteristics,
determine (S4) whether one or more of the primary signal characteristics and one or more of the secondary signal characteristics satisfy one or more pre-defined tolerance limits, and
retract (S5) the primary laser beam monitor (6) out of the treatment laser beam (T), if the one or more pre-defined tolerance limits are satisfied.

2. The ophthalmological laser treatment device (1) of claim 1, wherein the control module (3) is further configured to:
monitor (S6), continuously during treatment, using the secondary signal, the secondary signal characteristics, and determine whether the secondary signal characteristics satisfy one or more pre-defined operating criteria, and
stop (S7) the treatment laser beam (T) from exiting the application head (4), if the one or more secondary signal characteristics do not satisfy the pre-defined operating criteria.

3. The ophthalmological laser treatment device (1) of one of claims 1 or 2, wherein the control module (3) is further configured to:
monitor (S6), continuously during treatment, using the secondary signal, the secondary signal characteristics, the secondary signal characteristics including a beam position,
record (S8) the secondary signal characteristics during treatment, and
generate (S9) a dose map using the recorded secondary signal characteristics, the dose map indicative of the energy distributed in the eye (91).

4. The ophthalmological laser treatment device (1) of one of claims 1 to 3, wherein the primary laser beam monitor (6) comprises a photodiode and the primary signal characteristics include one or more of: a beam power, a pulse energy, or a beam position, and wherein the secondary laser beam monitor (7) comprises a photodetector array and the secondary signal characteristics include one or more of: a beam power, a pulse energy, a beam position, a beam orientation, or a beam profile.

5. The ophthalmological laser treatment device (1) of one of claims 1 to 4, wherein the control module (3) is configured to adjust (S10) the treatment laser beam (T), using one or more of: the primary signal characteristics or the secondary signal characteristics, by controlling one or more of: the treatment laser source (21), a laser attenuator (22), a beam shaper (23), or a scanner system (24).

6. The ophthalmological laser treatment device (1) of one of claims 1 to 5, wherein the secondary laser beam monitor (6) is arranged behind a beam-splitter (41).

7. The ophthalmological laser treatment device (1) of one of claims 1 to 6, wherein the primary laser beam monitor (6) comprises one or more of the following types of sensor device: a photodiode, a photodetector array, a thermopile, a position sensitive device, an optical power sensor, a microbolometer, or a pyroelectric detector.

8. The ophthalmological laser treatment device (1) of one of claims 1 to 7, wherein the secondary laser beam monitor (7) comprises one or more of the following types of sensor device: a photodiode, a photodetector array, a thermopile, a position sensitive device, an optical power sensor, a microbolometer, or a pyroelectric detector.

9. The ophthalmological laser treatment device (1) of one of claims 1 to 8, wherein a sensor device of the primary laser beam monitor (6) and a sensor device of the secondary laser beam monitor (7) have a different physical sensing principle.

10. The ophthalmological laser treatment device (1) of one of claims 1 to 9, wherein the base station (2) further comprises a pilot light source including one or more of: a pilot laser source or a pilot light-emitting diode, the pilot light source is coupled into the beam path, and wherein the secondary signal includes a pilot signal from the pilot light source.

11. The ophthalmological laser treatment device (1) of claim 10, wherein the secondary laser beam monitor (3) is arranged behind a beam-splitter (41) configured to partially reflect the treatment laser beam (T) and partially transmit the light from the light signal source.

12. The ophthalmological laser treatment device (1) of one of claims 1 to 11, wherein the primary signal characteristics and the secondary signal characteristics relate to one or more of the following properties of the treatment laser beam (T): a beam position, a beam orientation, a beam power, a pulse energy, or a beam profile.

13. The ophthalmological laser treatment device (1) of one of claims 1 to 12, wherein the control module (3) is further configured to generate an alarm message if at least one of the primary signal characteristics and at least one of the secondary signal characteristics do not satisfy the pre-defined tolerance limits.

14. A method for controlling an ophthalmological laser treatment device (1), the ophthalmological laser treatment device (1) comprising: a base station (2) having a treatment laser source (21) configured to generate a treatment laser beam (T), a control module (3), an application head (4), and an arm (5) arranged between the base station (2) and the application head (4) configured to provide a beam path for the treatment laser beam (T); wherein the application head (4) includes a primary laser beam monitor (6) which is retractable out of the treatment laser beam (T) and a secondary laser beam monitor (7), the method comprising:
moving (S1), by the control module (3), the primary laser beam monitor (6) into the treatment laser beam (T),
receiving (S2), in the control module (3), a primary signal from the primary laser beam monitor (6) and a secondary signal from the secondary laser beam monitor (7),
determining (S3), in the control module (3), using the primary signal, primary signal characteristics and determining, using the secondary signal, secondary signal characteristics,
determining (S4), in the control module (3), whether one or more of the primary signal characteristics and one or more of the secondary signal characteristics satisfy one or more pre-defined tolerance limits, and
retracting (S5), by the control module (3), the primary laser beam monitor (6) out of the treatment laser beam (T), if the one or more pre-defined tolerance limits are satisfied.
